# EUROPEAN PATENT SPECIFICATION

(11) **EP 1 651 227 B1**
(45) Date of publication and mention of the grant of the patent: **13.02.2008**
(21) Application number: 04743280.2
(22) Date of filing: 07.07.2004
(51) Int. Cl.: A61K 31/517, A61K 31/165, A61P 35/00

(54) **PHARMACEUTICAL COMPOSITIONS COMPRISING AZD 2171 AND ZD 6126 AND USES THEREOF**
PHARMAZEUTISCHE ZUBEREITUNGEN ENTHALTEND AZD 2171 UND ZD 6126 UND VERWENDUNGEN
COMPOSITIONS PHARMACEUTIQUES COMPRENANT DU AZD 2171 ET DU ZD 6126 ET LEURS UTILISATIONS

(30) Priority: 10.07.2003 GB 0316123
(43) Date of publication of application: 03.05.2006
(73) Proprietor: AstraZeneca AB, 151 85 Södertälje (SE)
(72) Inventor: WEDGE, Stephen Robert, AstraZeneca R & D Alderley, Macclesfield Cheshire SK10 4TG (GB)
(86) International application number: PCT/GB2004/002937
(87) International publication number: WO 2005/004871

(56) References cited:
- WO-A-00/47212

## Description

The present invention relates to a pharmaceutical composition comprising AZD2171 and ZD6126; to a combination product comprising AZD2171 and ZD6126 for use in a method of treatment of a human or animal body by therapy; to a kit comprising AZD2171 and ZD6126; to the use of AZD2171 and ZD6126 for the manufacture of a medicament for the production of an antiangiogenic and/or vascular permeability reducing effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor-A (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmernbrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fins-like tyrosine kinase receptor, Flt-1, the kinase insert domain-containing receptor, KDR (also referred to as Fllc-1), and another fins-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Application Publication No. WO 00/47212. In WO 00/47212 compounds are described which possess activity against VEGF receptor tyrosine kinase (VEGF RTK).

AZD2171 is 4-(4-fluoro-2-methylindol-5-yloxy)-6-methoxy-7-(3-(pyrrolidin-1-yl)propoxy)quinazoline:

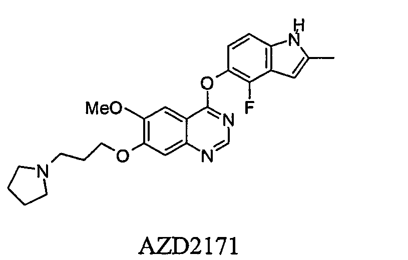

AZD2171 is Example 240 of WO 00/47212. AZD2171 is a very potent inhibitor of KDR and also has some activity against Flt-1. The IC₅₀ value for AZD2171 inhibition of KDR and Flt-1 tyrosine kinase activity, in recombinant enzyme assays *in vitro,* is < 2nM and 5nM respectively. AZD2171 has been shown to elicit broad-spectrum anti-tumour activity in a range of models following once-daily oral administration.

In WO 00/47212 it is stated that compounds of the invention: "may be applied as a sole therapy or may involve, in addition to a compound of the invention, one or more other substances and/or treatments. Such conjoint treatment may be achieved by way of the simultaneous, sequential or separate administration of the individual components of the treatment."

WO 00/47212 then goes on to describe examples of such conjoint treatment including surgery, radiotherapy and various types of chemotherapeutic agent including inhibitors of growth factor function and the vascular damaging agents described in International Patent Application Publication No. WO 99/02166 such as N-acetylcolchinol-O-phosphate. N-acetylcolchinol-O-phosphate is ZD6126.

Nowhere in WO 00/47212 is the specific combination of AZD2171 and ZD6126 suggested.

Nowhere in WO 00/47212 does it state that use of any compound of the invention therein with other treatments will produce surprisingly beneficial effects.

Angiogenesis produces neovascularisation in a number of disease states and reversal of neovascularisation by damaging the newly-formed vascular endothelium is expected to have a beneficial therapeutic effect. International Patent Application Publication No. WO 99/02166 describes tricyclic compounds that surprisingly have a selective damaging effect on newly formed vasculature as compared to the normal, established vascular endothelium of the host species. This is a property of value in the treatment of disease states associated with angiogenesis such as cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation including macular degeneration.

Compounds which damage newly formed vasculature are vascular targeting agents (VTAs) and are also known as vascular damaging agents (VDAs).

One such compound described in International Patent Application Publication No. WO 99/02166 is N-acetylcolchinol-O-phosphate, (also know as (5*S*)-5-(acetylamino)-9,10,11-trimethoxy-6,7-dihydro-5*H*-dibenzo[*a*,*c*]cyclohepten-3-yl dihydrogen phosphate; Example 1 of WO 99/02166), which is referred to herein as ZD6126:

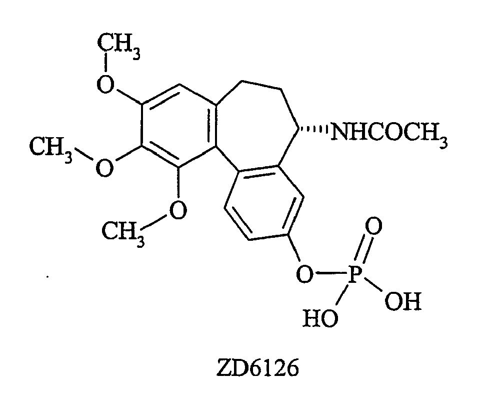

It is believed, though this is not limiting on the invention, that ZD6126 damages newly-formed vasculature, for example the vasculature of tumours, thus effectively reversing the process of angiogenesis. It has been reported that ZD6126 selectively disrupts tumour vasculature leading to vessel occlusion and extensive tumour necrosis (Davis PD, Hill SA, Galbraith SM, et al. Proc. Am. Assoc. Cancer Res. 2000; 41: 329).

In WO 99/02166 it is stated that: "compounds of the invention may be administered as sole therapy or in combination with other treatments. For the treatment of solid tumours compounds of the invention may be administered in combination with radiotherapy or in combination with other anti-tumour substances for example those selected from mitotic inhibitors, for example vinblastine, paclitaxel and docetaxel; alkylating agents, for example cisplatin, carboplatin and cyclophosphamide, antimetabolites, for example 5-fluorouracil, cytosine arabinoside and hydroxyurea; intercalating agents for example adriamycin and bleomycin; enzymes, for example asparaginase; topoisomerase inhibitors for example etoposide, topotecan and irinotecan; thymidylate synthase inhibitors for example raltitrexed; biological response modifers for example interferon; antibodies for example edrecolomab, and anti-hormones for example tamoxifen. Such combination treatment may involve simultaneous or sequential application of the individual components of the treatment."

Nowhere in WO 99/02166 does it suggest any combination of a VTA and a VEGF receptor tyrosine kinase inhibitor for the treatment of any disease state including cancer,

Nowhere in WO 99/02166 is the specific combination of ZD6126 and AZD2171 suggested.

Nowhere in WO 99/02166 does it state that use of any compound of the invention therein with other treatments will produce surprisingly beneficial effects,

Unexpectedly and surprisingly we have now found that the particular compound AZD2171 used in combination with a particular selection from the broad description of combination therapies listed in WO 00/47212, namely with ZD6126, produces significantly better effects than any one of AZD2171 and ZD6126 used alone. In particular, AZD2171 used in combination with ZD6126 produces significantly better effects on solid tumours than any one of AZD2171 and ZD6126 used alone.

Anti-cancer effects of a combination according to the present invention include anti-tumour effects, the response rate, the time to disease progression and the survival rate. Anti-tumour effects of a combination of the present invention include inhibition of tumour growth, tumour growth delay, regression of tumour, shrinkage of tumour, increased time to regrowth of tumour on cessation of treatment, slowing of disease progression. It is expected that when a combination of the present invention is administered to a warm-blooded animal such as a human, in need of treatment for cancer involving a solid tumour, said combination treatment will produce an effect, as measured by, for example, one or more of: the extent of the anti-tumour effect, the response rate, the time to disease progression and the survival rate. Anti-cancer effects include prophylactic treatment as well as treatment of existing disease.

According to an aspect of the invention there is provided a pharmaceutical composition which comprises AZD2171 or a pharmaceutically acceptable salt thereof, and ZD6126 or a pharmaceutically acceptable salt thereof, in association with a pharmaceutically acceptable excipient or carrier.

According to a further aspect of the present invention there is provided a combination product comprising AZD2171 or a pharmaceutically acceptable salt thereof and ZD6126 or a pharmaceutically acceptable salt thereof, for use in a method of treatment of a human or animal body by therapy,

According to a further aspect of the present invention there is provided a kit comprising AZD2171 or a pharmaceutically acceptable salt thereof, and ZD6126 or a pharmaceutically acceptable salt thereof.

According to the present invention AZD2171 or a pharmaceutically acceptable salt thereof and ZD6126 or a pharmaceutically acceptable salt thereof produce an antiangiogenic and/or vascular permeability reducing effect in a warm-blooded animal such as a human,

According to a further aspect of the present invention there is provided the use of AZD2171 or a pharmaceutically acceptable salt thereof in combination with ZD6126 or a pharmaceutically acceptable salt thereof for the manufacture of a medicament for the production of an anti-cancer effect in a warm-blooded animal such as a human.

According to a further aspect of the present invention there is provided the use of AZD2171 or a pharmaceutically acceptable salt thereof in combination with ZD6126 or a pharmaceutically acceptable salt thereof for the manufacture of a medicament for the production of an anti-tumour effect in a warm-blooded animal such as a human.

The use according to the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention.

Surgery may comprise the step of partial or complete tumour resection, prior to, during or after the administration of the combination treatment with AZD2171 described herein.

Other chemotherapeutic agents for optional use with a combination treatment of the present invention include those described in WO 00/47212 which is incorporated herein by reference. Such chemotherapy may cover five main categories of therapeutic agent:
(i) other antiangiogenic agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

The administration of a triple combination of AZD2171, ZD6126 and ionising radiation may produce effects, such as anti-tumour effects, greater than those achieved with any of AZD2171, ZD6126 and ionising radiation used alone, greater than those achieved with the combination of AZD2171 and ZD6126, greater than those achieved with the combination of AZD2171 and ionising radiation, greater than those achieved with the combination of ZD6126 and ionising radiation.

According to a further aspect of the present invention there is provided the use of AZD2171 or a pharmaceutically acceptable salt thereof in combination with ZD6126 or a pharmaceutically acceptable salt thereof for the manufacture of a medicament for the production of an antiangiogenic and/or vascular permeability reducing effect in a warm-blooded animal such as a human which is being treated with ionising radiation.

According to a further aspect of the present invention there is provided the use of AZD2171 or a pharmaceutically acceptable salt thereof in combination with ZD6126 or a pharmaceutically acceptable salt thereof for the manufacture of a medicament for the production of an anti-cancer effect in a warm-blooded animal such as a human which is being treated with ionising radiation.

According to a further aspect of the present invention there is provided the use of AZD2171 or a pharmaceutically acceptable salt thereof in combination with ZD6126 or a pharmaceutically acceptable salt thereof in the manufacture of a medicament for the production of an anti-tumour effect in a warm-blooded animal such as a human which is being treated with ionising radiation.

A warm-blooded animal such as a human which is being treated with ionising radiation means a warm-blooded animal such as a human which is treated with ionising radiation before, after or at the same time as the administration of a medicament or combination treatment comprising AZD2171 and ZD6126. For example said ionising radiation may be given to said warm-blooded animal such as a human within the period of a week before to a week after the administration of a medicament or combination treatment comprising AZD2171 and ZD6126. This means that AZD2171, ZD6126 and ionising radiation may be administered separately or sequentially in any order, or may be administered simultaneously. The warm-blooded animal may experience the effect of each of AZD2171, ZD6126 and radiation simultaneously.

According to one aspect of the present invention the ionising radiation is administered before one of AZD2171 and ZD6126 or after one of AZD2171 and ZD6126.

According to one aspect of the present invention the ionising radiation is administered before both AZD2171 and ZD6126 or after both AZD2171 and ZD6126.

According to one aspect of the present invention AZD2171 is administered to a warm-blooded animal after the animal has been treated with ionising radiation.

In another aspect of the present invention AZD2171 is dosed daily continuously for a longer period of time during which time ZD6126 and ionising radiation are each administered periodically, that is for a few days, for example 1,2, 3, 4 or 5 days at a time.

According to another aspect of the present invention the effect of a combination treatment of the present invention is expected to be at least equivalent to the addition of the effects of each of the components of said treatment used alone, that is, of each of AZD2171 and ZD6126 used alone or of each of AZD2171, ZD6126 and ionising radiation used alone.

According to another aspect of the present invention the effect of a combination treatment of the present invention is expected to be greater than the addition of the effects of each of the components of said treatment used alone, that is, of each of AZD2171 and ZD6126 used alone or of each of AZD2171, ZD6126 and ionising radiation used alone.

According to another aspect of the present invention the effect of a combination treatment of the present invention is expected to be a synergistic effect.

A combination treament is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with AZD2171 or ZD6126 or ionising radiation alone. Further, the effect of the combination treatment is synergistic if a beneficial effect is obtained in a group of patients that does not respond (or responds poorly) to AZD2171 or ZD6126 or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of AZD2171 or ZD6126 or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments are of interest for their antiangiogenic (including vascular damaging) and/or vascular permeability effects. Angiogenesis, neovascularisation and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, endometriosis, dysfunctional uterine bleeding and ocular diseases with retinal vessel proliferation including age-related macular degeneration. Combination treatments of the present invention are expected to be particularly useful in the prophylaxis and treatment of diseases such as cancer and Kaposi's sarcoma. In particular such combination treatments of the invention are expected to slow advantageously the growth of primary and recurrent solid tumours of, for example, the colon, breast, prostate, lungs and skin. In one aspect of the present invention such combination treatments of the invention are expected to slow advantageously the growth of primary and recurrent solid tumours of the breast. In one aspect of the present invention such combination treatments of the invention are expected to slow advantageously the growth of primary and recurrent solid tumours of the lung, for example in non-small cell lung cancer (NSCLC). In one aspect of the present invention such combination treatments of the invention are expected to slow advantageously the growth of primary and recurrent solid tumours of the kidney. In one aspect of the present invention such combination treatments of the invention are expected to slow advantageously the growth of primary and recurrent solid tumours of the colon and/or rectum.

In another aspect of the present invention AZD2171 and ZD6126, optionally with ionising radiation, are expected to inhibit the growth of those primary and recurrent solid tumours which are associated with VEGF especially those tumours which are significantly dependent on VEGF for their growth and spread. Combination treatments of the invention are expected to inhibit any form of cancer associated with VEGF including leukaemia, mulitple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), kidney, breast, prostate, lung, vulva and skin, particularly NSCLC.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the AZD2171 and ZD6126 of the combination treatment may be delivered endoscopically, intratracheally, intralesionally, percutaneously, intravenously, subcutaneously, intraperitoneally or intratumourally. Preferably AZD2171 is administered orally. Preferably ZD6126 is administered intravenously. In general the compositions described herein may be prepared in a conventional manner using conventional excipients. The compositions of the present invention are advantageously presented in unit dosage form.

AZD2171 will normally be administered to a warm-blooded animal at a unit dose within the range 1-50mg per square metre body area of the animal, for example approximately 0.03-1.5 mg/kg in a human. A unit dose in the range, for example, 0.01-1.5mg/kg, preferably 0.03-0.5mg/kg is envisaged and this is normally a therapeutically-effective dose. A unit dosage form such as a tablet or capsule will usually contain, for example 1-50mg of active ingredient. Preferably a daily dose in the range of 0.03-0.5mg/kg is employed.

ZD6126 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-250mg of active ingredient. Preferably a daily dose in the range of 0.5-5mg/kg is employed.

It has been reported, in International Patent Application Publication No. WO 01/74369, that the effect of a given dose of ZD6126 can be increased by administering it in divided doses. Divided doses, also called split doses, means that the total dose to be administered to a warm-blooded animal, such as a human, in any one day period (for example one 24 hour period from midnight to midnight) is divided up into two or more fractions of the total dose and these fractions are administered with a time period between each fraction of about greater than 0 hours to about 10 hours, preferably about 1 hour to about 6 hours, more preferably about 2 hours to about 4 hours. The fractions of total dose may be about equal or unequal.

For example the total dose may be divided into two parts which may be about equal with a time interval between doses of greater than or equal to two hours and less than or equal to 4 hours.

ZD6126 may be administered in divided doses when used in combination with AZD2171.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1 Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity. The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

The present invention relates to combinations of ZD6126 or a salt thereof with AZD2171 or with a salt of AZD2171.

Salts of ZD6126 for use in pharmaceutical compositions will be pharmaceutically acceptable salts, but other salts may be useful in the production of ZD6126 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6126 may be made according to the following process.

N-Acetylcolchinol (30.0g, 83.9mmol) is dissolved in acetonitrile under an inert atmosphere and 1,2,3-triazole (14.67g, 212.4mmol) added via a syringe. Di-tert-butyldiethylphosphoramidite (37.7g, 151.4mmol) is added and the reaction mixture stirred at about 20°C to complete the formation of the intermediate phosphite ester. Cumene hydroperoxide (24.4g, 159.2mmol) is added at about 10°C and the reaction mixture stirred until the oxidation is complete. Butyl acetate (50ml) and sodium hydroxide solution (250ml of 1M) are added, the reaction mixture stirred and the aqueous phase discarded. The organic solution is washed with sodium hydroxide solution (2 x 250ml of 1M) and a saturated solution of sodium chloride. Trifluoroacetic acid (95.3g, 836mmol) is added at about 15°C. The reaction mixture is distilled at atmospheric pressure, ZD6126 crystallises and is isolated at ambient temperature.

Salts of AZD2171 for use in pharmaceutical compositions will be pharmaceutically acceptable salts, but other salts may be useful in the production of AZD2171 and its pharmaceutically acceptable salts. Pharmaceutically acceptable salts may, for example, include acid addition salts. Such acid addition salts include for example salts with inorganic or organic acids affording pharmaceutically acceptable anions such as with hydrogen halides or with sulphuric or phosphoric acid, or with trifluoroacetic, citric or maleic acid. In addition pharmaceutically acceptable salts may be formed with an inorganic or organic base which affords a pharmaceutically acceptable cation. Such salts with inorganic or organic bases include for example an alkali metal salt, such as a sodium or potassium salt and an alkaline earth metal salt such as a calcium or magnesium salt.

AZD2171 may be synthesised according to the processes described in WO 00/47212, in particular those described in Example 240 of WO 00/47212.

The following tests may be used to demonstrate the activity of AZD2171 in combination with ZD6126.

### Human LoVo colorectal carcinoma tumour xenografts in Nude mice

1 x 10⁷ LoVo tumour cells in 0.1 ml of serum free Dulbecco's Modified Eagle's Medium (DMEM) were injected subcutaneously (s.c.) into the flank of each athymic *(nu*/*nu* genotype) mouse. Tumour volumes were assessed by bilateral Vernier caliper measurement and, taking length to be the longest diameter across the tumour and width the corresponding perpendicular, calculated using the formula (length x width) x the square root of (length x width) x (π/6). Five days after implantation (referred to as "day 0"), when tumours reached a mean volume of 0.8cm³, mice were randomised into groups of nine and treated with AZD2171 (3 mg/kg/day orally, day 0 to day 14), ZD6126 (100 mg/kg/day i.p., day 0 to day 2), or a combination thereof with either "concurrent administration" (AZD2171 day 0 - day 14 combined with ZD6126 day 0 - day 2, where AZD2171 was dosed 2 hours prior to ZD6126) or "sequential administration" (ZD6126 day 0 - day 2 followed by AZD2171 day 3 - day 14). AZD2171, was dosed orally at 0.1ml/10g body weight, as a suspension in 1% polysorbate 80 (i.e. a 1% (v/v) solution of polyoxyethylene (20) sorbitan mono-oleate in deionised water). ZD6126 was dissolved in a solution of 0.05% sodium carbonate in physiological saline and administered by intraperitoneal injection at 0.1ml/10g body weight. Control animals received AZD2171 vehicle throughout the experiment (orally, day 0 - day 14). Inhibition of tumour growth from the start of treatment was assessed on day 14 by comparison of the differences in tumor volume between control and treated groups. In addition, the number of tumour regressions following 14 days of treatment was ascertained (tumour regression being evident if the tumour volume at day 14 was smaller than the pre-treatment value on day 0).

**Table I**

| Treatment | Mean % Inhibition of tumour growth at day 14 (P value by two-tailed t-test) | Number of tumour regressions evident at day 14 |
|---|---|---|
| AZD2171 (3 mg/kg/day, days 0 -14, p.o.) | 62 % (P< 0.001) | 0/9 |
| ZD6126 (100 mg/kg/day, days 0 - 2, i.p.) | 65 % (P< 0.001) | 1/9 |
| ZD6126 (day 0 - 2) + AZD2171 (day 3 - 14) "Sequential schedule" | 96 % (P<0.001) | 2/9 |
| AZD2171 (day 0 - 14) + ZD6126 (day 0 - 2) "Concurrent schedule" | 160 % (P<0.001) (i.e. 60 % tumour regression) | 9/9 |

The combination of AZD2171 with ZD6126 produced a greater inhibition of tumour growth than each of AZD2171 and ZD6126 alone. Tumour volumes at day 14 following sequential combination therapy (ZD6126 day 0-2 followed by AZD2171 day 3-14) were significantly smaller than those evident after treatment with AZD2171 (day 0-14) or ZD6126 (day 0-2) alone (P<0.001, two-tailed t-test). Tumour volumes at day 14 following concurrent combination therapy (AZD2171 day 0-14 combined with ZD6126 day 0-2) were also significantly smaller than those evident after treatment with AZD2171 or ZD6126 alone (P < 0.0001; two-tailed t-test) and significantly smaller than those following sequential combination treatment (P< 0.0001; two-tailed t-test). Regression was evident in all LoVo colorectal carcinoma xenografts at day 14 when the concurrent AZD2171 + ZD6126 combination schedule was used.

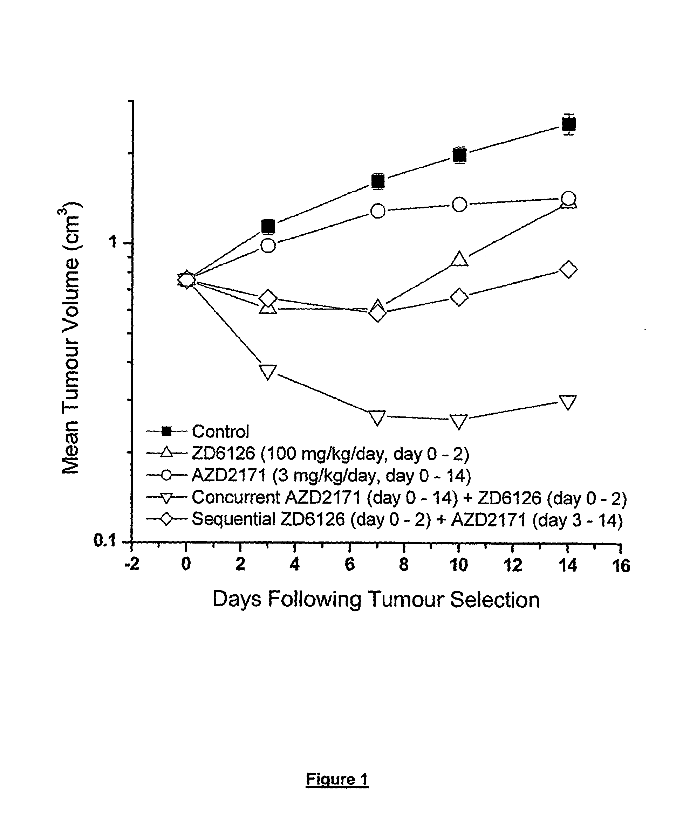

The data is shown graphically in Figure 1.

## Claims

1. Use of AZD2171 or a pharmaceutically acceptable salt thereof in combination with ZD6126 or a pharmaceutically acceptable salt thereof for the manufacture of a medicament for the production of an anti-cancer effect in a warm-blooded animal such as a human.

2. Use of AZD2171 or a pharmaceutically acceptable salt thereof in combination with ZD6126 or a pharmaceutically acceptable salt thereof for the manufacture of a medicament for the production of an anti-cancer effect in a warm-blooded animal such as a human which is being treated with ionising radiation.

3. Use of AZD2171 or a pharmaceutically acceptable salt thereof in combination with ZD6126 or a pharmaceutically acceptable salt thereof for in the manufacture of a medicament for the production of an anti-tumour effect in a warm-blooded animal such as a human.

4. Use of AZD2171 or a pharmaceutically acceptable salt thereof in combination with ZD6126 or a pharmaceutically acceptable salt thereof for the manufacture of a medicament for the production of an anti-tumour effect in a warm-blooded animal such as a human which is being treated with ionising radiation.

5. Use according to claim 1 or claim 2 wherein the cancer is a cancer of the breast, lung, kidney or colon including rectum.

6. Use according to claim 3 or claim 4 wherein the tumour is a tumour of the breast, lung, kidney or colon including rectum.

7. A pharmaceutical composition which comprises AZD2171 or a pharmaceutically acceptable salt thereof, and ZD6126 or a pharmaceutically acceptable salt thereof, in association with a pharmaceutically acceptable excipient or carrier.

8. A kit comprising AZD2171 or a pharmaceutically acceptable salt thereof, and ZD6126 or a pharmaceutically acceptable salt thereof.

## Patentansprüche

1. Verwendung von AZD2171 oder einem pharmazeutisch annehmbaren Salz davon in Kombination mit ZD6126 oder einem pharmazeutisch annehmbaren Salz davon zur Herstellung eines Arzneimittels zur Erzielung einer Antikrebswirkung bei einem Warmblüter wie einem Menschen.

2. Verwendung von AZD2171 oder einem pharmazeutisch annehmbaren Salz davon in Kombination mit ZD6126 oder einem pharmazeutisch annehmbaren Salz davon zur Herstellung eines Arzneimittels zur Erzielung einer Antikrebswirkung bei einem Warmblüter wie einem Menschen, der mit ionisierender Strahlung behandelt wird.

3. Verwendung von AZD2171 oder einem pharmazeutisch annehmbaren Salz davon in Kombination mit ZD6126 oder einem pharmazeutisch annehmbaren Salz davon zur Herstellung eines Arzneimittels zur Erzielung einer Antitumorwirkung bei einem Warmblüter wie einem Menschen.

4. Verwendung von AZD2171 oder einem pharmazeutisch annehmbaren Salz davon in Kombination mit ZD6126 oder einem pharmazeutisch annehmbaren Salz davon zur Herstellung eines Arzneimittels zur Erzielung einer Antitumorwirkung bei einem Warmblüter wie einem Menschen, der mit ionisierender Strahlung behandelt wird.

5. Verwendung nach Anspruch 1 oder 2, bei der es sich bei dem Krebs um einen Krebs der Brust, der Lunge, der Niere oder des Kolons einschließlich des Rektums handelt.

6. Verwendung nach Anspruch 3 oder 4, bei der es sich bei dem Tumor um einen Tumor der Brust, der Lunge, der Niere oder des Kolons einschließlich des Rektums handelt.

7. Pharmazeutische Zusammensetzung, enthaltend AZD2171 oder ein pharmazeutisch annehmbares Salz davon und ZD6126 oder ein pharmazeutisch annehmbares Salz davon zusammen mit einem pharmazeutisch annehmbaren Hilfsstoff oder Träger.

8. Kit, enthaltend AZD2171 oder ein pharmazeutisch annehmbares Salz davon und ZD6126 oder ein pharmazeutisch annehmbares Salz davon.

## Revendications

1. Utilisation d'AZD2171 ou d'un sel de celui-ci, acceptable d'un point de vue pharmaceutique, en combinaison avec du ZD6126 ou un sel de celui-ci, acceptable d'un point de vue pharmaceutique, pour la fabrication d'un médicament destiné à la production d'un effet anticancéreux chez un animal à sang chaud tel qu'un être humain.

2. Utilisation d'AZD2171 ou d'un sel de celui-ci, acceptable d'un point de vue pharmaceutique, en combinaison avec du ZD6126 ou un sel de celui-ci, acceptable d'un point de vue pharmaceutique, pour la fabrication d'un médicament destiné à la production d'un effet anticancéreux chez un animal à sang chaud, tel qu'un être humain, qui est traité par rayonnement ionisant.

3. Utilisation d'AZD2171 ou d'un sel de celui-ci, acceptable d'un point de vue pharmaceutique, en combinaison avec du ZD6126 ou un sel de celui-ci, acceptable d'un point de vue pharmaceutique, pour la fabrication d'un médicament destiné à la production d'un effet antitumoral chez un animal à sang chaud tel qu'un être humain.

4. Utilisation d'AZD2171 ou d'un sel de celui-ci, acceptable d'un point de vue pharmaceutique, en combinaison avec du ZD6126 ou un sel de celui-ci, acceptable d'un point de vue pharmaceutique, pour la fabrication d'un médicament destiné à la production d'un effet antitumoral chez un animal à sang chaud, tel qu'un être humain, qui est traité par rayonnement ionisant.

5. Utilisation selon la revendication 1 ou la revendication 2, dans laquelle le cancer est un cancer du sein, du poumon, du rein ou du côlon, y compris du rectum.

6. Utilisation selon la revendication 3 ou la revendication 4, dans laquelle la tumeur est une tumeur du sein, du poumon, du rein ou du côlon, y compris du rectum.

7. Composition pharmaceutique qui comprend de l'AZD2171 ou d'un sel de celui-ci, acceptable d'un point de vue pharmaceutique, et du ZD6126 ou un sel de celui-ci, acceptable d'un point de vue pharmaceutique, en association avec un excipient ou véhicule acceptable d'un point de vue pharmaceutique.

8. Kit comprenant de l'AZD2171 ou un sel de celui-ci, acceptable d'un point de vue pharmaceutique, et du ZD6126 ou un sel de celui-ci, acceptable d'un point de vue pharmaceutique.
